# EUROPEAN PATENT APPLICATION

(11) **EP 4 371 578 A1**
(43) Date of publication of application: **22.05.2024**
(21) Application number: 22842492.5
(22) Date of filing: 14.07.2022
(51) Int. Cl.: A61K 47/61, A61K 9/00, A61P 17/00, A61K 31/575

(54) **PHARMACEUTICAL COMPOSITION COMPRISING HYALURONIC ACID COMPLEX FOR PREVENTION OR TREATMENT OF SKIN DISEASE**

(30) Priority: 15.07.2021 KR 20210092915
(71) Applicant: Ajou University Industry-Academic Cooperation Foundation, Gyeonggi-do 16499 (KR)
(72) Inventor: KIM, Wook, Suwon-si Gyeonggi-do 16509 (KR); KIM, Eun Ha, Seoul 02794 (KR); LEE, Wang Hee, Anyang-si Gyeonggi-do 14096 (KR); LEE, Seul Bi, Seoul 05605 (KR); YOON, Ju Hwan, Suwon-si Gyeonggi-do 16692 (KR); YU, So Hee, Gwangju-si Gyeonggi-do 12773 (KR); SONG, Yu Jin, Busan 47902 (KR); CHOI, Hong Seo, Gunpo-si Gyeonggi-do 15875 (KR); YANG, Ye Young, Buyeo-gun Chungcheongnam-do 33219 (KR); KIM, Su Ha, Daejeon 34061 (KR); RHO, Jun Gi, Ansan-si Gyeonggi-do 15596 (KR); HAN, Ji Hye, Suwon-si Gyeonggi-do 16689 (KR); KWEON, So Hui, Suwon-si Gyeonggi-do 16245 (KR)
(74) Representative: J A Kemp LLP
(86) International application number: PCT/KR2022/010310
(87) International publication number: WO 2023/287230

(57) **Abstract**

The present invention relates to a pharmaceutical composition for preventing or treating inflammatory skin disease comprising a hyaluronic acid conjugate as an active ingredient. The hyaluronic acid conjugate has a structure in which hyaluronic acid and a hydrophobic substance providing a hydrophobic group are linked together by self-assembly. The hyaluronic acid conjugate has excellent resistance to hyaluronidase, excellent transdermal permeability, and excellent effects on the protection or restoration of skin barrier function, the inhibition of expression of cell proliferation-related factors and inflammatory cytokines, the inhibition of M1 macrophage polarization, and the blockage of TLR4 signaling, and thus may be used as a therapeutic agent for various inflammatory skin diseases.

## Description

### Technical Field

The present invention relates to a pharmaceutical composition for preventing or treating skin disease comprising a hyaluronic acid conjugate as an active ingredient.

### Background Art

This application was requested by the Korea Drug Development Fund and was conducted as part of the new drug-based expansion research of National New Drug Development Project. The research project name of this application is `Securing a lead material for the treatment of skin inflammatory diseases through the optimization-profiling-establishment of production technology for hyaluronic acid nanoparticles that control immune cell activity', and the project number is RS-2021-DD120838 (formerly HN21C0958).

Methods of administering drugs may be broadly divided into oral administration and transdermal administration. Thereamong, transdermal administration has several advantages over oral administration in order to apply drugs locally, particularly to the skin. For example, an orally administered drug is degraded to a significant extent by liver metabolism, and thus when a drug is to be administered orally, an excessive amount of the drug needs to be administered to obtain a certain drug effect and needs to be repeatedly administered to maintain the drug effect. On the other hand, transdermal drug delivery may solve the disadvantages that may occur due to oral administration because the transdermal administered drug is delivered directly to the affected area in a controlled manner without undergoing liver metabolism. In addition, transdermal administration has advantages in that it is possible to reduce patient's pain and psychological burden, which are caused upon administration by i.v. injection, and in that there is no discomfort. Therefore, transdermal administration is suggested as an effective dosage method for treating skin diseases that occur mainly locally and occur in the skin and its surrounding tissue.

Since the skin is primarily composed of the epidermis, dermis, and subcutaneous fat, and the skin tissue is dense, percutaneous absorption of a drug may be difficult or severe skin irritation may occur, depending on the molecular weight, dose, and on characteristics of the drug, which may limit the choice of drugs. Therefore, for effective transdermal administration, nanoparticulated drugs may be used or various drug delivery systems that help deliver drugs from the skin surface to the tissue under the skin may be used. Recently, drug delivery systems for transdermal administration including various biodegradable polymers such as polylactic acid, polyglycolic acid, and hyaluronic acid have been developed.

Hyaluronic acid is a linear polysaccharide polymer having a molecular weight ranging from 1×10⁵ to 1×10⁷ Da, and consists of repeating units of (β,1-4)-D-glucuronic acid (GlcUA) and (β,1-3)-N-acetyl-D-glucosamine (GlcNAc). Hyaluronic acid is found in the extracellular matrix and cell surfaces of most human tissues and is particularly present in large amounts in synovial fluid and cartilage. Accordingly, hyaluronic acid has biocompatibility and is biodegraded by hyaluronidase in blood, and thus it is used as biomaterials such as drug delivery systems and scaffolds for tissue engineering. In particular, hyaluronic acid binds to CD44 and RHAMM, which are overexpressed on the surfaces of cancer cells or metastatic cancer cells, is internalized through endocytosis, and is degraded in low-pH environments such as lysosomes. In addition, hyaluronic acid plays an important role as a signaling molecule in cell motility, cell differentiation, wound healing, and cancer metastasis.

### DISCLOSURE

### Technical Problem

Linear hyaluronic acid has problems in that it has a low skin absorption rate due to its molecular weight is too large to penetrate the skin, so there has been no example of the application of transdermal administration of hyaluronic acid to treat diseases, and in that hyaluronic acid is easily degraded by hyaluronidase *in vivo,* making it difficult to sustain the medicinal effect thereof. Therefore, there is an urgent need for efforts to overcome these problems and to develop a hyaluronic acid-containing drug or drug delivery system that is stable *in vivo* and has increased percutaneous permeability so that hyaluronic acid may be applied to transdermal administration.

### Technical Solution

An object of the present invention is to provide a pharmaceutical composition for preventing or treating skin disease comprising, as an active ingredient, a hyaluronic acid conjugate in which a hydrophobic substance is linked with hyaluronic acid.

### Advantageous Effects

The hyaluronic acid conjugate according to the present invention has a structure in which hyaluronic acid and a hydrophobic substance providing a hydrophobic group are linked together by self-assembly. The hyaluronic acid conjugate has excellent resistance to hyaluronidase and transdermal permeability, and has excellent effects on the protection and recovery of skin barrier function, the inhibition of expression of cell proliferation-related factors and inflammatory cytokines, the inhibition of M1 macrophage polarization, and the blocking of TLR4 signaling, and thus may be used as an agent for treating various skin diseases.

### Brief Description of Drawings

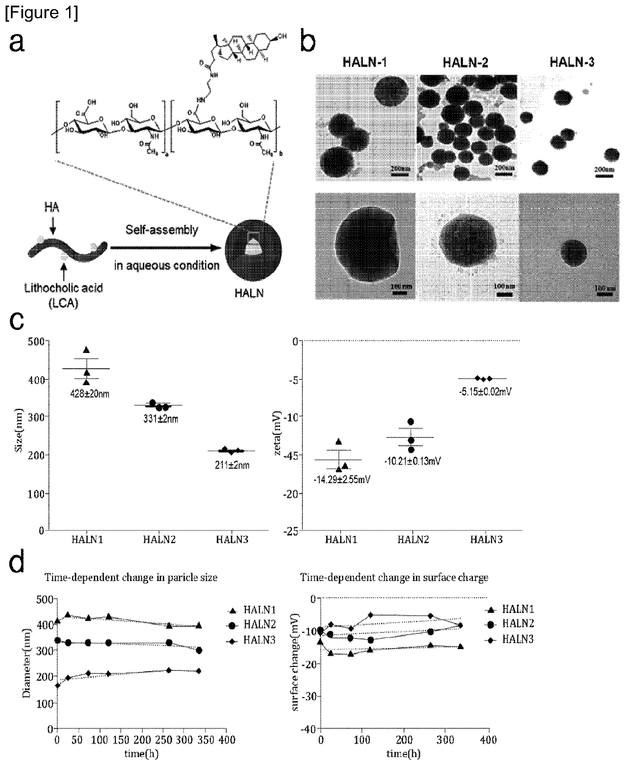

FIG. 1 shows the self-assembly characteristics of hyaluronic acid-lithocholic acid conjugates (HALN-1, HALN-2 and HALN-3). Specifically, FIG. 1(a) is a schematic view showing the chemical structure and self-assembly of the hyaluronic acid-lithocholic acid conjugate, FIG. 1(b) shows transmission electron microscope (TEM) images of the hyaluronic acid-lithocholic acid conjugates, FIG. 1(c) shows the sizes and zeta potentials of the hyaluronic acid-lithocholic acid conjugate, and FIG. 1(d) shows graphs about stability obtained by measuring time-dependent changes in particle size and zeta potential.
FIG. 2 shows the resistance of the hyaluronic acid-lithocholic acid conjugates (HALN-1, HALN-2 and HALN-3) to hyaluronidase.
FIG. 3 shows the psoriasis treatment effect of the hyaluronic acid-lithocholic acid conjugates (HALN-1, HALN-2 and HALN-3). Specifically, FIG. 3(a) shows the experimental schedule, and FIG. 3(b) shows the skin conditions of mice with erythema and scaling and the PASI scores of the mice.
FIG. 4 shows the skin thickness and inflammatory cytokine ((IL-1β and IL-23) expression levels measured by histological staining of mouse skin in order to evaluate the psoriasis treatment effect of the hyaluronic acid-lithocholic acid conjugates (HALN-1, HALN-2 and HALN-3).
FIG. 5 shows the expression levels of skin barrier function-related markers (ceramide, loricrin, claudin-1, and S100A9) measured by histological staining of mouse skin in order to evaluate the skin barrier function protective effect of the hyaluronic acid-lithocholic acid conjugates (HALN-1, HALN-2 and HALN-3).
FIG. 6 shows the skin barrier function recovery effect of the hyaluronic acid-lithocholic acid conjugate (HALN-3).
FIG. 7 is a graph showing the fluorescence intensity of Cy5.5-HALN, measured to evaluate the transdermal permeability of the hyaluronic acid-lithocholic acid conjugate (HALN-3) through the Franz diffusion cell.
FIG. 8 depicts images showing the fluorescence intensity of Cy5.5-HALN in mouse skin tissue, measured to evaluate the transdermal permeability of the hyaluronic acid-lithocholic acid conjugate (HALN-3) in normal mice and mice with skin disease (psoriasis).
FIG. 9 shows fluorescence intensity depending on the mouse skin tissue depth, measured to evaluate the transdermal permeability of the hyaluronic acid-lithocholic acid conjugate (HALN-3).
FIG. 10 shows the transdermal permeability of the hyaluronic acid-lithocholic acid conjugates (HALN-1, HALN-2 and HALN-3).
FIG. 11 shows the psoriasis treatment effect of transdermal administration of the hyaluronic acid-lithocholic acid conjugate (HALN-3) at various concentrations (0.5, 2.5, and 5 mg). Specifically, FIG. 11(a) shows the experimental schedule, and FIG. 11(b) shows the skin conditions of mice with erythema and scaling and the PASI scores of the mice.
FIG. 12 shows the skin thickness and the expression levels of a cell proliferation related factor (Ki-67) and inflammatory cytokines (CD68, IL-23 and IL-17), measured by histological staining of mouse skin in order to evaluate the psoriasis treatment effect of transdermal administration of the hyaluronic acid-lithocholic acid conjugate (HALN-3) at various concentrations (0.5, 2.5, and 5 mg).
FIG. 13 shows the psoriasis preventive effect of transdermal administration of the hyaluronic acid-lithocholic acid conjugate (HALN-3) at various concentrations (2 and 10 mg). Specifically, FIG. 13(a) shows the experimental schedule, and FIG. 13(b) shows the skin conditions of mice with erythema and scaling and the PASI scores of the mice.
FIG. 14 shows the skin thickness and the expression levels of a cell proliferation related factor (Ki-67) and inflammatory cytokines (CD68, IL-23 and IL-17), measured by histological staining of mouse skin in order to evaluate the psoriasis preventive effect of transdermal administration of the hyaluronic acid-lithocholic acid conjugate (HALN-3) at various concentrations (2 and 10 mg).
FIG. 15 shows the expression levels of skin barrier function-related markers (ceramide, loricrin, claudin-1 and S100A9) measured by histological staining of mouse skin in order to evaluate the skin barrier function protective effect of transdermal administration of the hyaluronic acid-lithocholic acid conjugate (HALN-3).
FIG. 16 shows the macrophage polarization inhibitory effect of HALN with various sizes. Specifically, FIG. 16(a) shows the expression levels of M1 macrophage polarization markers (TNF-α, IL-6, IL-1β, IL-12p40, and IL-23p19) by HALN-1 to 3, and FIG. 16 (b) shows the polarization level of M1 macrophages by HALN-3.
FIG. 17 shows a transmission electron microscope (TEM) image and chemical formula of a hyaluronic acid-cholanic acid conjugate (HACN).
FIG. 18 shows the fluorescence intensity of Cy5.5-HACN measured in mouse skin tissue in order to evaluate the transdermal permeability of the hyaluronic acid-cholanic acid conjugate.
FIG. 19 shows the macrophage-dependent psoriasis treatment effect of the hyaluronic acid-cholanic acid conjugate. Specifically, FIG. 19(a) shows the experimental schedule, and FIG. 19(b) shows the skin conditions of mice with erythema and scaling and the PASI scores of the mice.
FIG. 20 shows the macrophage-dependent psoriasis treatment effect of the hyaluronic acid-cholanic acid conjugate. Specifically, FIGS. 20(a) and 20(b) show the skin thickness and the expression level of skin cell proliferation-related factor (Ki-67), respectively, measured by histological staining of non-clodronate-treated mouse skin.
FIG. 21 shows the expression levels of inflammatory cytokines (IL-1β, IL-23, and IL-17) measured in mouse skin with or without clodronate treatment in order to evaluate the macrophage-dependent psoriasis treatment effect of the hyaluronic acid-cholanic acid conjugate.
FIG. 22 shows the macrophage polarization inhibitory effect of HACN with various sizes. Specifically, FIG. 22(a) shows the expression levels of polarization markers (TNF-α, IL-6, COX-2, IL-12p40, IL-23p19, and CXCL10) of M1 macrophages by HACN, and FIG. 22(b) shows the polarization level of M1 macrophages by HACN.
FIG. 23 shows the interaction between HACN and TLR4, measured through surface plasmon resonance.
FIG. 24 shows the competition between LPS and HACN for binding to TLR4, measured through fluorescence cytometry.
FIGS. 25 and 26 show the results of evaluating the effects of various types of hyaluronic acid conjugates on the treatment of skin diseases.

### Mode for Invention

The present inventors have conducted studies to develop a hyaluronic acid-containing drug or drug delivery system that is stable *in vivo* and has excellent transdermal permeability, and as a result, have found that a nano-sized hyaluronic acid conjugate fabricated by self-assembling low-molecular-weight hyaluronic acid, which is a biodegradable hydrophilic substance, with a hydrophobic substance such as lithocholic acid, cholanic acid or cholesterol, has excellent *in vivo* stability and transdermal permeability and is effective in alleviating and treating symptoms of skin diseases such as psoriasis, thereby completing the present invention.

One aspect of the present invention provides a pharmaceutical composition for preventing or treating skin disease comprising, as an active ingredient, a hyaluronic acid conjugate composed of hyaluronic acid and a hydrophobic substance.

As used herein, the term "hydrophobic substance" refers to a compound containing a hydrophobic substituent, which is a substance imparting hydrophobicity to hydrophilic hyaluronic acid. Specifically, the hydrophobic substance may be a substance having hydrophobicity capable of inducing the formation of a nano-sized hyaluronic acid conjugate.

According to one embodiment of the present invention, the hydrophobic substance may have a partition coefficient (LogP) of 2 to 10, preferably 3 to 8.

The hydrophobic substance having such a partition coefficient value may form a nano-sized hyaluronic acid conjugate.

According to one embodiment of the present invention, the hydrophobic substance may induce the hyaluronic acid conjugate to have a particle size of 50 to 500 nm.

The hydrophobic substance according to the present invention preferably has biodegradable properties that are easily degraded by enzymes produced *in vivo* or degraded during metabolism *in vivo.*

More specifically, the hydrophobic substance may be hydrophobic cholanic acid, which is a type of bile acid, or a derivative thereof.

According to one embodiment of the present invention, the hydrophobic substance may be at least one selected from the group consisting of lithocholic acid, cholanic acid, cholic acid, chenodeoxycholic acid, glycocholic acid, taurocholic acid, deoxycholic acid, 7-oxo-lithocholic acid, and cholesterol.

According to one embodiment of the present invention, the hyaluronic acid conjugate may be one in which hyaluronic acid and a hydrophobic substance are linked together by an amide bond.

In one example of the present invention, in order to bind a hydrophobic substance to hyaluronic acid, lithocholic acid or cholanic acid and ethylenediamine were allowed to react with each other to produce an aminated hydrophobic substance, and the amine group of the hydrophobic substance was bonded to the carboxyl group of hyaluronic acid to form a hyaluronic acid conjugate.

In one example of the present invention, in order to bind a hydrophobic substance to hyaluronic acid, cholesterol was allowed to react with DCC or the like to produce an aminated hydrophobic substance, and the amine group of the hydrophobic material was bonded to the carboxyl group of hyaluronic acid to form a hyaluronic acid conjugate.

According to one embodiment of the invention, the hyaluronic acid conjugate may be a compound represented by Formula 1, Formula 2 or Formula 3 below:

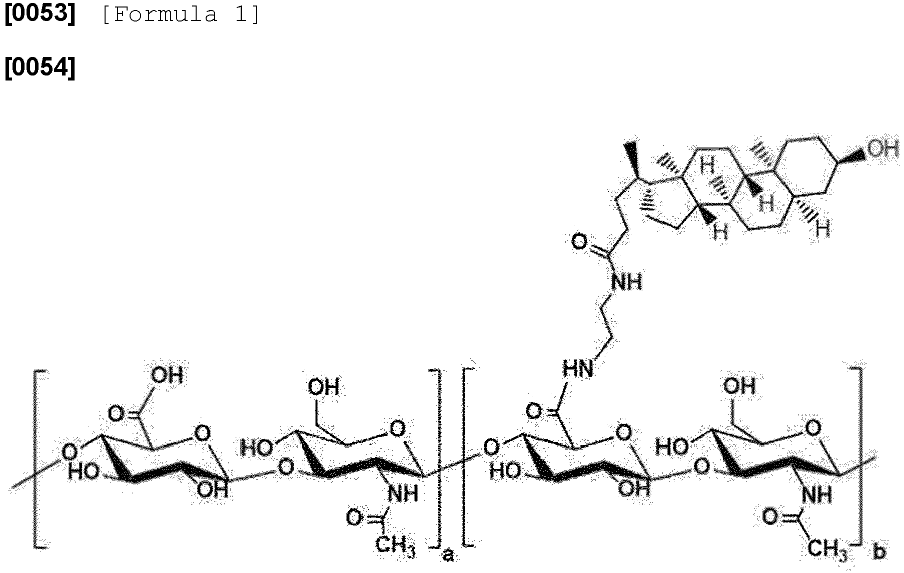

In Formula 1 above, a and b are independent integers that are the same as or different from each other, a is an integer ranging from 1 to 750, b is an integer ranging from 1 to 100, and b/(a+b) is in the range of 0.01 to 0.40.

More specifically, in Formula 1 above, a is an integer ranging from 1 to 300 (preferably an integer ranging from 1 to 150), and b is an integer ranging from 1 to 40 (preferably an integer ranging from 1 to 20), and b/(a+b) may be 0.01 to 0.30 (preferably 0.02 to 0.20).

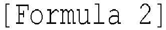

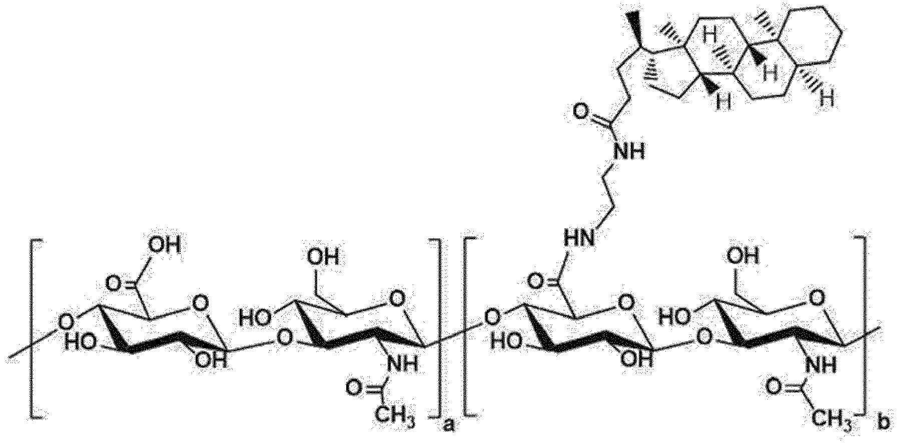

In Formula 2, a and b are independent integers that are the same as or different from each other, a is an integer ranging from 1 to 750, b is an integer ranging from 1 to 100, and b/(a+b) is in the range of 0.01 to 0.40.

More specifically, in Formula 2 above, a is an integer ranging from 1 to 100 (preferably an integer ranging from 1 to 30), b is an integer ranging from 1 to 10 (preferably 1 or 2), and b/(a+b) may be 0.01 to 0.20 (preferably 0.02 to 0.10).

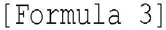

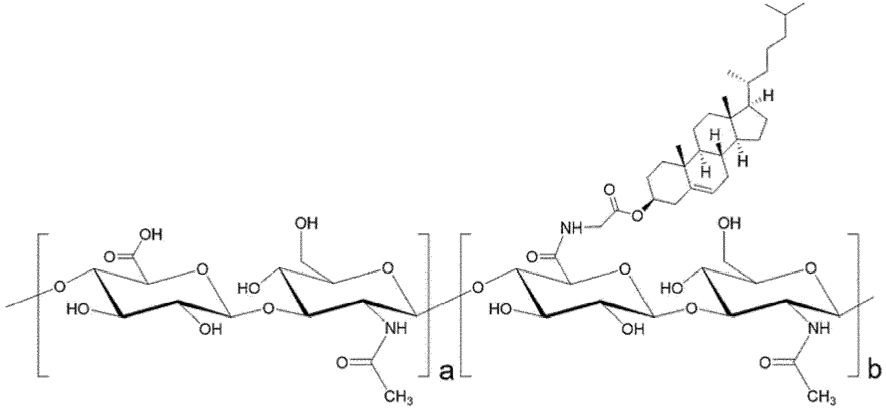

In Formula 3 above, a and b are independent integers that are the same as or different from each other, a is an integer ranging from 1 to 750, and b is an integer ranging from 1 to 100, and b/(a+b) is in the range of 0.01 to 0.40.

More specifically, in Formula 3 above, a is an integer ranging from 1 to 300 (preferably an integer ranging from 1 to 150), b is an integer ranging from 1 to 40 (preferably an integer ranging from 1 to 20), and b/(a+b) may be 0.01 to 0.30 (preferably 0.02 to 0.20).

According to one embodiment of the present invention, the mass ratio of hyaluronic acid to the hydrophobic substance in the hyaluronic acid conjugate may be 1:0.005 to 0.5.

More specifically, when the hydrophobic substance in the hyaluronic acid conjugate is lithocholic acid or cholanic acid, the mass ratio of hyaluronic acid or cholanic acid to lithocholic acid may be 1: 0.005 to 0.5, 1: 0.01 to 0.3, or 1: 0.02 to 0.15.

General linear hyaluronic acid has a high molecular weight (about 1×10⁵ to 1×10⁷ Da), and thus is not able to easily penetrate the skin. In the present invention, hyaluronic acid that has a low molecular weight so as to be easily absorbed into the skin is preferably used.

According to one embodiment of the present invention, the hyaluronic acid conjugate may have a molecular weight of 1 to 300 kDa.

More specifically, the number average molecular weight of the hyaluronic acid conjugate is preferably 10 to 250 kDa, more preferably 40 to 80 kDa, for Formula 1, is preferably from 1 to 50 kDa, more preferably from 5 to 30 kDa, for Formula 2, and is preferably 1 to 50 kDa, more preferably 5 to 30 kDa, for Formula 3.

According to one embodiment of the present invention, the hyaluronic acid conjugate may form nanoparticles by self-assembly in an aqueous solution.

According to one embodiment of the present invention, the hyaluronic acid conjugate may be in the nanoparticles having a diameter of 50 to 500 nm.

According to one embodiment of the present invention, the degree of substitution (DS; the number of hydrophobic moieties attached per 100 sugar residues in hyaluronic acid) may vary depending on the amount of hydrophobic substance adjusted according to the feed ratio (FR). The degree of substitution of the hydrophobic substance may be an integer ranging from 1 to 50, preferably an integer ranging from 2 to 15.

In one example of the present invention, it was confirmed that, as the degree of substitution (DS) of the hydrophobic group in the hyaluronic acid conjugate increased, the size of the hyaluronic acid conjugate nanoparticles decreased. It was confirmed that, as the size of the hyaluronic acid conjugate nanoparticles decreased, the hyaluronic acid conjugate nanoparticles had increased resistance to hyaluronidase, increased transdermal permeability, and better effects on the inhibition of expression of skin proliferation-related factors and inflammatory cytokines. Therefore, in the present invention, the hyaluronic acid conjugate nanoparticles preferably have a diameter of 100 to 400 nm, more preferably 150 to 300 nm. If the size of the hyaluronic acid nanoparticles is larger than the upper limit of the above range, the transdermal permeability of the hyaluronic acid conjugate may decrease, and thus the effect of the hyaluronic acid conjugate on the prevention or treatment of skin diseases may be reduced.

According to one embodiment of the present invention, the hyaluronic acid conjugate may be for transdermal administration.

In one example of the present invention, it was confirmed that the hyaluronic acid conjugate had an excellent ability to penetrate skin tissue or to be absorbed into skin tissue, and thus was absorbed into deeper skin tissue than linear hyaluronic acid. Therefore, it can be seen that the hyaluronic acid conjugate according to the present invention is suitable for transdermal administration.

According to one embodiment of the present invention, the skin disease may be at least one selected from the group consisting of atopic dermatitis, acne, psoriasis, allergic dermatitis, inflammatory skin disease, seborrheic dermatitis, contact dermatitis, scleroderma, eczema, Behcet's disease, sarcoidosis, melanoma, vitiligo, pemphigus, corns, warts, and lichen planus.

In one example of the present invention, it was confirmed that the hyaluronic acid conjugate improved skin conditions by reducing erythema and scaling on the skin of psoriasis-induced mice, restored damaged skin barrier function, and inhibited the expression of the skin proliferation-related factor Ki-67 and the inflammatory cytokines CD68, IL-23 and IL-17.

In addition, it can be seen that the hyaluronic acid conjugate may bind to TLR4 and block TLR4 signaling, thereby preventing or treating various skin diseases caused by TLR4 signaling.

The pharmaceutical composition comprising the hyaluronic acid conjugate as an active ingredient may further comprise a pharmaceutically acceptable additive. Examples of the additive include, but are not limited to, starch, gelatinized starch, microcrystalline cellulose, lactose, povidone, colloidal silicon dioxide, hydroxy calcium phosphate, lactose, mannitol, maltose, gum Arabic, pregelatinized starch, corn starch, powdered cellulose, hydroxypropyl cellulose, Opadry, starch sodium glycolate, lead carnauba, synthetic aluminum silicate, stearic acid, magnesium stearate, aluminum stearate, calcium stearate, white sugar, dextrose, sorbitol, and talc.

According to one embodiment of the present invention, the pharmaceutical composition may further comprise a pharmaceutically acceptable carrier. Examples of the carrier include, but are not limited to, lactose, dextrose, sucrose, sorbitol, mannitol, trehalose, hyaluronic acid, starch, gum Acacia, calcium phosphate, alginate, gelatin, calcium silicate, microcrystalline cellulose, polyvinylpyrrolidone, cellulose, water, syrup, methylcellulose, methyl hydroxybenzoate, propyl hydroxybenzoate, talc, magnesium stearate, and mineral oil, which are commonly used in the manufacture of medicaments.

According to one embodiment of the present invention, the pharmaceutical composition may further comprise a lubricant, a wetting agent, a sweetening agent, a flavoring agent, an emulsifying agent, a suspending agent, a preservative, and the like. Suitable pharmaceutically acceptable carriers and formulations are described in detail in Remington's Pharmaceutical Sciences (22th ed., 2013).

The pharmaceutical composition of the present invention may be administered in various oral and parenteral dosage forms during actual clinical administration, and may be formulated with commonly used diluents or excipients, such as fillers, extenders, binders, wetting agents, disintegrants, surfactants, etc. Solid formulations for oral administration include tablets, pills, powders, granules, capsules and the like, and such solid formulations may be prepared by mixing the pharmaceutical composition of the present invention with at least one excipient, for example, starch, calcium carbonate, sucrose, lactose, or gelatin. In addition to simple excipients, lubricants such as magnesium stearate or talc may also be used.

The pharmaceutical composition of the present invention may be administered orally or parenterally depending on the desired method. For parenteral administration, transdermal administration, intraperitoneal injection, intrarectal injection, subcutaneous injection, intravenous injection, intramuscular injection, or intrathoracic injection may be selected. The dosage varies depending on the patient's weight, age, sex, health condition, diet, administration time, administration mode, excretion rate, and the severity of the disease.

The pharmaceutical composition of the present invention is administered in a pharmaceutically effective amount. As used herein, the term "pharmaceutically effective amount" refers to an amount sufficient to treat a disease at a reasonable benefit/risk ratio applicable to any medical treatment. The effective dose level of the pharmaceutical composition may be determined depending on factors, including the kind and severity of the patient's disease, the activity of the drug, sensitivity to the drug, the time of administration, the route of administration, excretion rate, the duration of treatment, and drugs used in combination with the composition, as well as other factors well known in the medical field. The pharmaceutical composition according to one embodiment of the present invention may be administered individually or in combination with other therapeutic agents, and may be administered sequentially or simultaneously with conventional therapeutic agents. The pharmaceutical composition may be administered in a single or multiple dosage form. It is important to administer the pharmaceutical composition in the minimum amount that can exhibit the maximum effect without causing side effects, in view of all the above-described factors, and this amount can be easily determined by a person skilled in the art.

Specifically, the effective amount of the pharmaceutical composition of the present disclosure may vary depending on the patient's age, sex and body weight. Generally, the pharmaceutical composition may be administered daily or every other day at a dose of 0.001 to 1,000 mg/kg body weight, 0.01 to 100 mg/kg body weight, or 0.1 to 10 mg/kg body weight, or may be administered 1 to 3 times a day at this dose. However, the dose is not intended to limit the scope of the present invention in any way, because the dose may increase or decrease depending on the route of administration, the severity of the disease, the patient's sex, weight and age, etc.

Another aspect of the present invention provides a method for preventing or treating skin disease comprising a step of administering to a subject a composition comprising, as an active ingredient, a hyaluronic acid conjugate composed of hyaluronic acid and a hydrophobic substance.

According to one embodiment of the present invention, in the method, details regarding the hyaluronic acid, the hydrophobic substance, the hyaluronic acid conjugate, and the skin disease are as described above.

According to one embodiment of the present invention, the dose of the composition may be 10 mg/kg/day to 100 mg/kg/day.

According to one embodiment of the present invention, the subject may be a subject in need of prevention or treatment of skin disease. The subject may be selected from among mammals, such as primates, including humans and monkeys, or rodents, including mice and rats, cells or tissues isolated therefrom, or cultures thereof. In this case, the patient may be a mammal other than a human.

Still another aspect of the present invention provides the use of a hyaluronic acid conjugate composed of hyaluronic acid and a hydrophobic substance for the prevention or treatment of skin disease.

Yet another aspect of the present invention provides the use of a hyaluronic acid conjugate composed of hyaluronic acid and a hydrophobic substance for the manufacture of a medicament for the prevention or treatment of skin disease.

According to one embodiment of the present invention, in the use, details regarding the hyaluronic acid, the hydrophobic substance, the hyaluronic acid conjugate, and the skin disease are as described above.

### Mode for Invention

Hereinafter, the present invention will be described in more detail. However, this description is merely an example intended to help understand the present invention, and the scope of the present invention is not limited by this exemplary description.

### Example 1. Production of hyaluronic acid-lithocholic acid conjugate

### 1-1. Amination of lithocholic acid

1 g of lithocholic acid (LCA) (Sigma-Aldrich) was dissolved in 20 mL of methanol, and then 180 µL of HCl (12 N) was added thereto, followed by stirring at 70° C for 8 hours under reflux conditions. After completion of the reaction, the solvent was distilled off under reduced pressure, and the resulting solid product was diluted in water, and the precipitate was collected by filtration under reduced pressure and dried in a vacuum to finally obtain lithocholic acid methyl ester. 920 mg of the LCA methyl ester was dissolved in 15 mL of ethylenediamine (TCI) and stirred at 130° C for 8 hours under reflux conditions. After completion of the reaction, the ethylenediamine was distilled out under reduced pressure, and the resulting product was diluted in water, and the precipitate was collected by filtration under reduced pressure and dried to finally obtain aminated LCA.

### 1-2. Hyaluronic acid-lithocholic acid conjugate (HALN)

120 mg of 60 kDa hyaluronic acid (HA; Lifecore)) was dissolved in 20 mL of formamide (Sigma-Aldrich). Then, 1-ethyl-3-(3-dimethylaminopropyl)carbodiimide (EDC; TCI) (56.1 mg, 0.29 mmol) and N-hydroxysuccinimide (NHS; Sigma) (33.3 mg, 0.29 mmol) were added thereto, followed by stirring for 30 minutes, thus preparing an HA mixture. An appropriate amount of aminated LCA (8 to 80 mg) according to the feed ratio (FR) value was dissolved in 5 mL of dimethylformamide (DMF), and then this solution was added dropwise to the HA mixture. The resulting mixture was stirred at room temperature for 24 hours. After completion of the reaction, the reaction mixture was dialyzed using a 13K MWCO seamless cellulose tubing dialysis bag (Sigma-Aldrich) in methanol alone for 2 days, a mixture of methanol: distilled water (v:v=1:1) for 1 day, and distilled water alone for 2 days. After completion of dialysis, the reaction mixture was sonicated for 50 seconds (10 seconds on, and 1 second off). After sonication, the mixture was filtered through a 0.8-um syringe filter. The filtrate was freeze-dried to finally obtain a hyaluronic acid-lithocholic acid conjugate (HALN). Next, the HALN was analyzed by nuclear magnetic resonance (NMR) spectroscopy and classified into HALN-1, HALN-2 and HALN-3 based on the degree of substitution (DS; the number of hydrophobic moieties).

### 1-3. Characterization of HALN

Chemical characterization of HALN was performed by nuclear magnetic resonance (NMR) spectroscopy at 600 MHz using deuterated methanol: deuterium oxide (1:1 (v:v) as a solvent. The size and zeta potential of the produced HALN were measured using dynamic light scattering (DLS) (ELS-Z, Otsuka). For measurement, HALN was dissolved in PBS at a concentration of 1 mg/mL.

As a result, as shown in FIG. 1, when the dried HALN was dispersed in PBS, functional groups of lithocholic acid aggregated together to form a spherical shape. HALN-1, HALN-2, and HALN-3, whose degree of substitution (DS) increased as the feed rate (FR) increased, were synthesized. It was confirmed that the sizes of HALN-1, HALN-2 and HALN-3 were 428120 nm, 331±2 nm and 211±2 nm, respectively, and as the DS increased, the sizes were smaller in the order of HALN-1, HALN-2 and HALN-3 (about 150 to 440 nm), and the zeta potential value increased.

### Experimental Example 1. Analysis of resistance of HALN to hyaluronidase

Each of HALN-1, HALN-2 and HALN-3, which have different sizes and electrochemical values, was allowed to react with hyaluronidase type II (H2126, Sigma) at 37°C for 0.5, 1, 2, 4 and 6 hours, and then the reaction was stopped by heating at 100°C. Each reaction product was treated sequentially with potassium tetraborate and DMAB solution, followed by incubation in a water bath at 37°C. 200 µl of each reaction solution was loaded into a 96-well plate, and the absorbance was measured at 544 nm with a spectrophotometer (Cytation 3, BioTek Instruments Inc.).

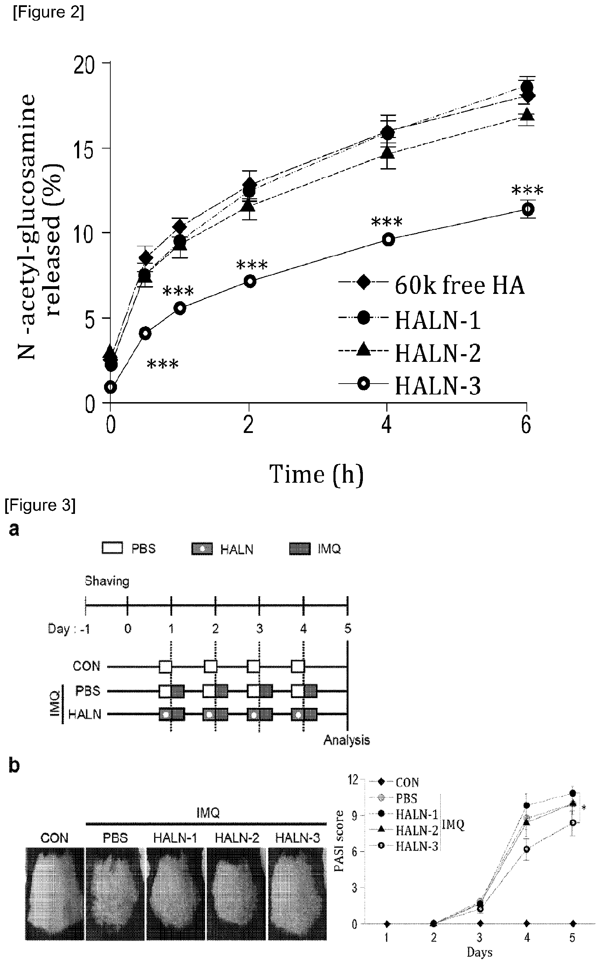

As a result, as shown in FIG. 2, it was confirmed that HALN-3 has significantly higher resistance to hyaluronidase than hyaluronic acid alone (60 kDa linear HA) and the other conjugates (HALN-1 and HALN-2).

### Experimental Example 2. Psoriasis treatment effect of HALN

Female C57BL/6N mice (OrientBio) were used to prepare psoriasis animal models (mice per group). The mice were divided into normal mice (CON) and psoriasis-induced mice (IMQ). Before inducing psoriasis, each of HALN-1 to 3 was dissolved in PBS at a concentration of 1 mg/mL, and then subcutaneously injected at a dose of 20 mg/kg for 4 days using an insulin syringe, or PBS (20 mg/kg) instead of HALN was administered alone. Two hours after drug administration, the body weight and PASI score of each mouse were measured. In addition, after drug administration, 62.5 mg of Aldara cream (imiquimod, IMQ) was applied to the dorsal area of each shaved mouse once daily for 4 days. On day 5, skin tissues were collected from all of the mice and analyzed.

The PASI scores were determined based on the psoriasis area and severity as follows: 0; none, 1; slight, 2; moderate, 3; substantial, and 4; very well pronounced.

For histological staining of mouse skin, each tissue was fixed with 4% paraformaldehyde (PFA), and then a paraffin block was made and sectioned into 4-pm-thick slides. Next, the nucleus and cytoplasm were stained with hematoxylin and eosin. Subsequently, staining with IL-1β and IL-23 antibodies was performed to analyze the expression of inflammatory cytokines.

The skin thickness was automatically analyzed using the 'MRI Skin Tool' function of the Image J program. The average of three partial image values for each tissue was used.

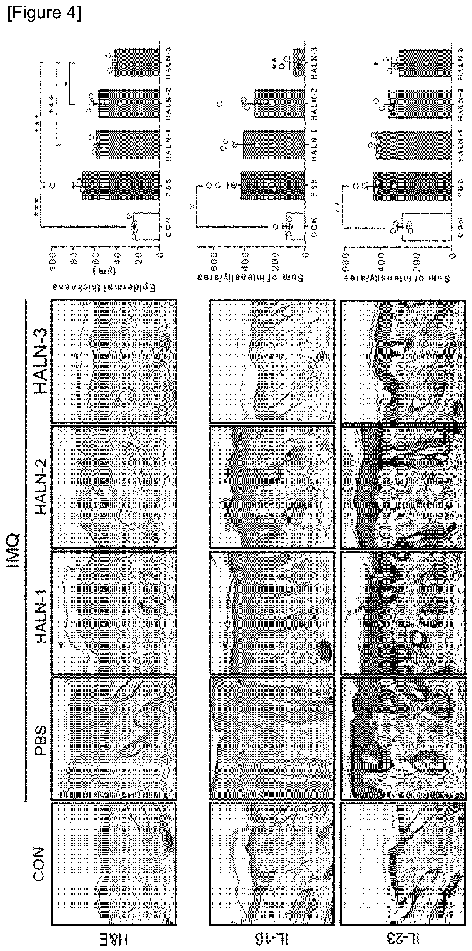

As a result, as shown in FIGS. 3 and 4, it was confirmed that, when HALN-1 to 3 were administered, erythema, scaling, and skin thickness were reduced compared to when PBS was administered, and in particular, HALN-3 having the smallest size had the best effect. In addition, it was confirmed that HALN-3 effectively inhibited the expression of IL-1β and IL-23, which are important inflammatory cytokines in psoriasis, compared to HALN-1 and HALN-2. In conclusion, HALN-3 had the best effect on the prevention or treatment of psoriasis compared to HALN-1 and HALN-2.

### Experimental Example 3. Protective effect of HALN on skin barrier function

Female C57BL/6N mice were used to prepare psoriasis animal models (five mice per group). The mice were divided into normal mice (CON) and psoriasis-induced mice (IMQ). Before inducing psoriasis, each of HALN-1 to 3 was dissolved in PBS at a concentration of 1 mg/mL, and then subcutaneously injected to the dorsal area of each shaved mouse at a dose of 20 mg/kg using an insulin syringe, or PBS instead of HALN was administered alone at 20 mg/kg. Three hours after drug administration, 62.5 mg of Aldara cream was applied for 4 days. On the last day of administration, skin tissues were collected from all of the mice and immunostained with markers (ceramide, loricrin, claudin-1, and S100A9) representing skin barrier function.

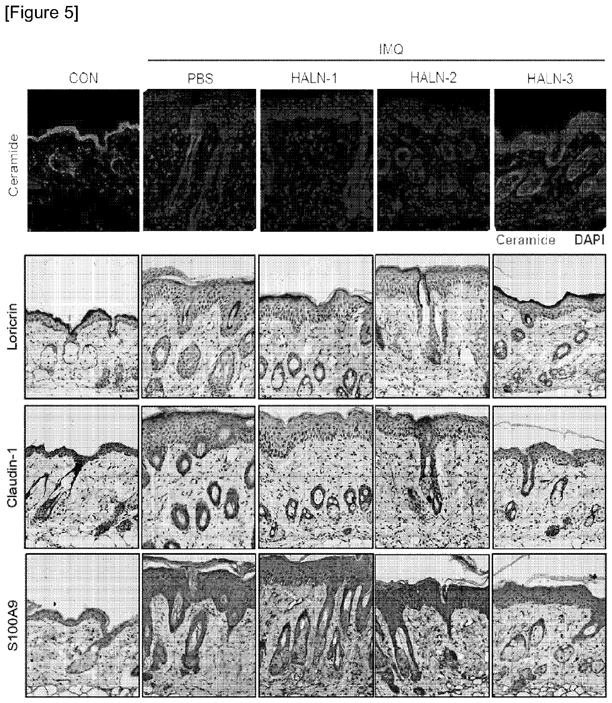

As a result, as shown in FIG. 5, it was confirmed that HALN-3 had the best effect on the restoration of the skin barrier function destroyed by psoriasis induction compared to HALN-1 and HALN-2.

In addition, after PBS or HALN-3 was administered to the psoriasis animal models in the same manner as in the above experiment, Cy5.5-HALN-3 was transdermally administered to all of the mice on the last day, and the skin tissues were observed under a fluorescence microscope 4 hours later.

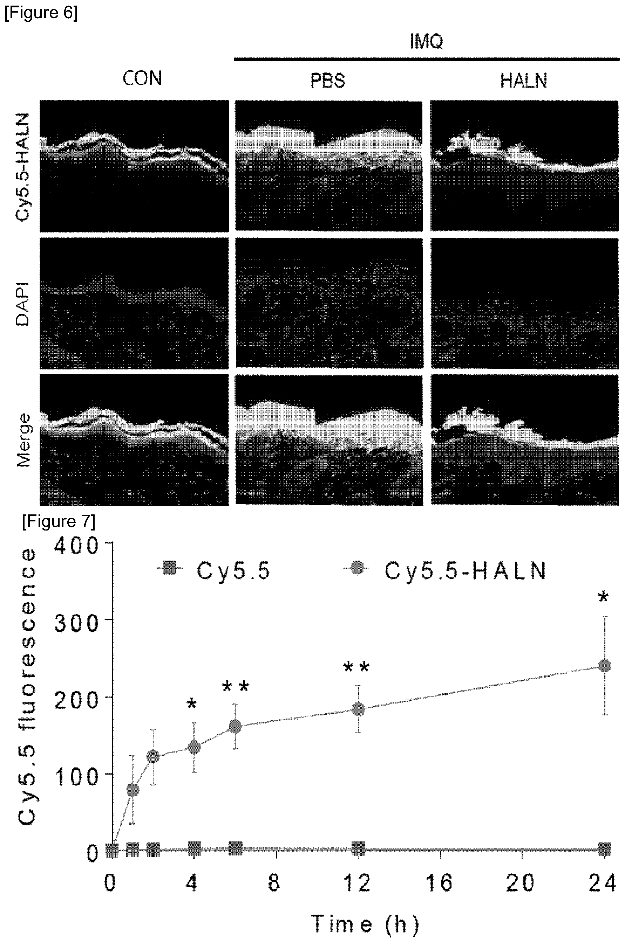

**As** a result, as shown in FIG. 6, it could be seen that the percutaneous absorption of Cy5.5-HALN-3 in the psoriasis animal model injected with HALN-3 was less than that in the psoriasis animal model injected with PBS, suggesting that HALN-3 restored the skin barrier function destroyed by psoriasis induction.

### Experimental Example 4. Analysis of transdermal permeability of HALN

### 4-1. Franz diffusion cell

After the skin tissue derived from a micropig was fixed to a Franz diffusion cell, Cy5.5-HALN-3 (0.5 ml) was applied onto the skin tissue. At 0, 1, 2, 4, 6, 12, and 24 hours after application, 20 µl of the solution was extracted from the recipient chamber and loaded into a black 96-well plate, and fluorescence (absorption wavelength: 670 nm, and emission wavelength: 700 nm) was measured using a spectrophotometer (Cytation 3, BioTekInstruments Inc.). Cy5.5 was used as a control.

As a result, as shown in FIG. 7, it could be seen that Cy5.5-HALN that penetrated the skin tissue increased with the passage of time, indicating that HALN had transdermal permeability.

In addition, after each of the dorsal skin from PBS-applied normal mice and the dorsal skin from mice in which psoriasis was induced as described in Experimental Example 3 was fixed to a Franz diffusion cell, Cy5.5-HALN (0.5 ml) was applied to each of the skin tissues. At 1, 2, 4 and 12 hours after application, the skin tissues were collected, and each tissue was fixed with 4% PFA. Then, a paraffin block was made and sectioned into 4-µm-thick slides, thereby preparing samples for histological staining. In addition, after DAPI staining, the skin tissues were observed under a fluorescence microscope.

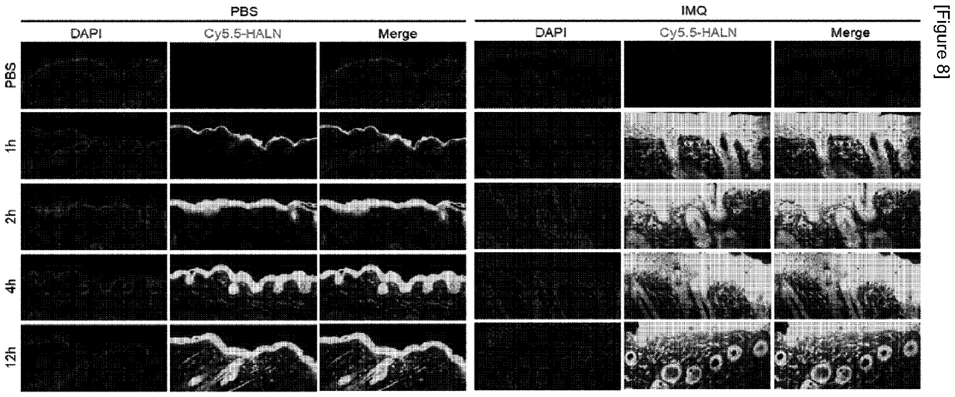

As a result, as shown in FIG. 8, it was confirmed that the fluorescence intensity was stronger in the psoriasis-induced mice (IMQ) than in the normal mice under the same time conditions, suggesting that the transdermal permeability of HALN in the psoriasis skin was better.

In addition, the dorsal skin from mice in which psoriasis was induced as described in Experimental Example 3 was fixed to a Franz diffusion cell, and then PBS, Cy5.5 (0.5 ml), Cy5.5-HA (0.5 ml) or Cy5.5-HALN (0.5 ml) was applied onto the skin tissue. Four hours after application, the skin tissues were collected and made into histologically stained samples, and then fluorescence was observed by two-photon microscopy.

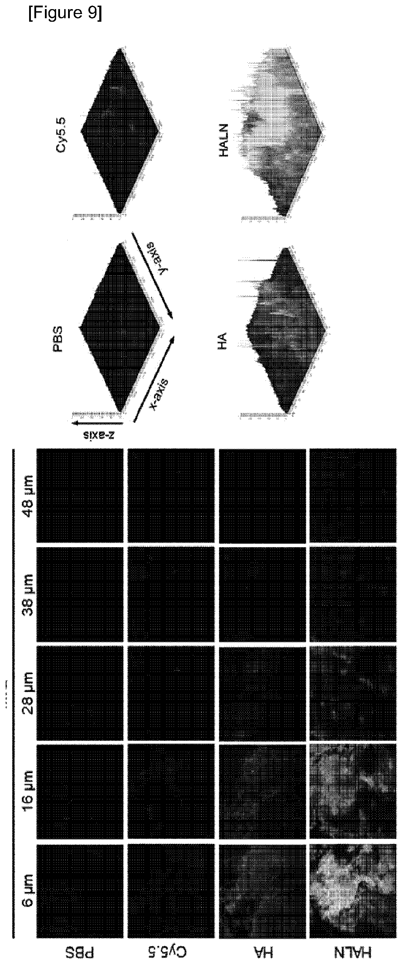

As a result, as shown in FIG. 9, HALN showed a stronger fluorescence intensity than Cy5.5 and free HA at the same depth of skin tissue, and the fluorescence thereof was observed even at a deeper depth. These results suggest that HALN has excellent transdermal permeability.

### 4-2. Comparison of transdermal permeability of HALN-1 to 3

Each of Cy5.5-HALN-1 to 3 was applied to the dorsal skin of each of PBS-applied normal mice and mice in which psoriasis was induced as described in Experimental Example 3. Four hours after application, the skin tissues were collected and observed under a fluorescence microscope.

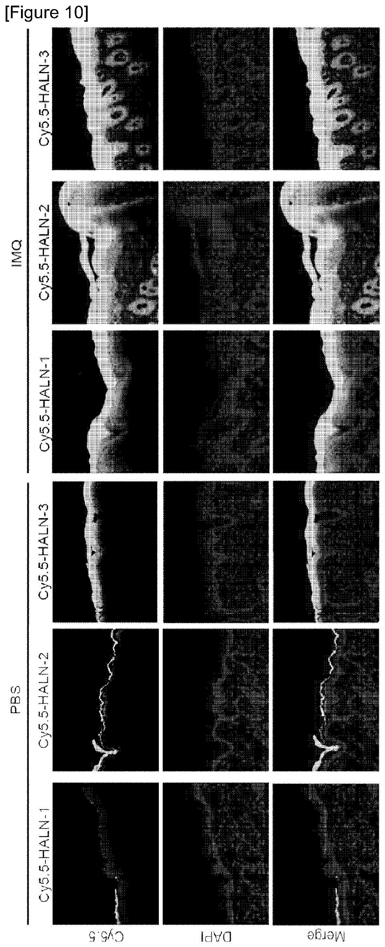

As a result, as shown in FIG. 10, it was confirmed that the transdermal permeability of all the HALN in the psoriasis animal models increased compared to that in the normal mice, and in particular, the transdermal permeability of HALN-3 was the best.

### Experimental Example 5. Concentration-dependent effect of transdermal administration of HALN on treatment and prevention of psoriasis

Female C57BL/6N mice were used to prepare psoriasis animal models (five mice per group) . The mice were divided into normal mice (CON) and psoriasis-induced mice (IMQ). To induce psoriasis, 62.5 mg of Aldara cream was applied to the dorsal area of each shaved mouse once daily for 4 days. From day 2 of application, 3 hours before administration of Aldara cream, PBS (20 mg/kg) or HALN-3 (0.5, 2.5, or 5 mg) was applied once daily for 3 days using a brush. On day 5, skin tissues and spleens were collected from all of the mice.

In the same manner as in Experimental Example 2, the PASI scores were determined, and histological staining was performed to measure the skin thickness and the expression of cell proliferation-related factors (Ki-67 and CD68) and inflammatory cytokines (IL-23 and IL-17).

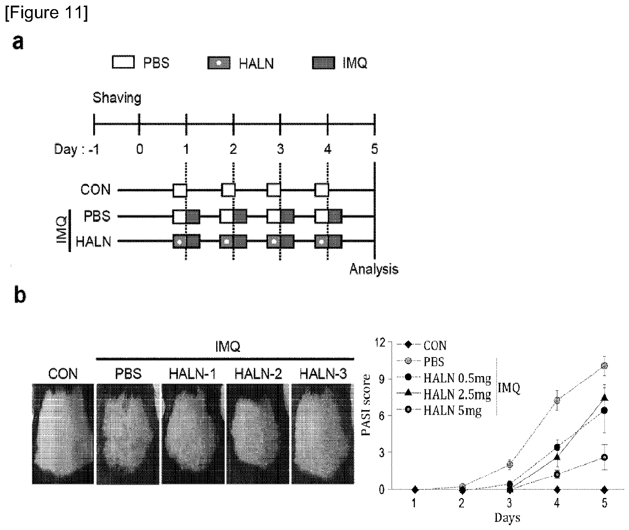

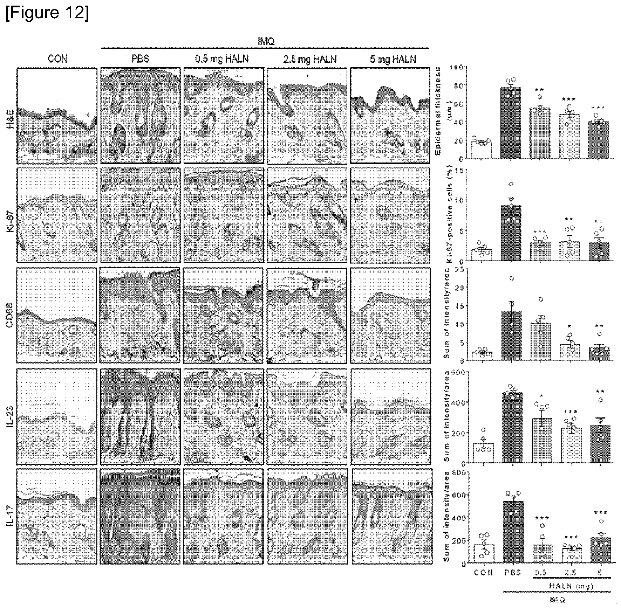

As a result, as shown in FIGS. 11 and 12, it was confirmed that, when HALN was administered, the skin condition was improved in a concentration-dependent manner, and cell proliferation-related factors and inflammatory cytokines were effectively inhibited.

These results suggest that HALN is effective in treating skin inflammatory diseases such as psoriasis.

Female C57BL/6N mice were used to prepare psoriasis animal models (five mice per group). The mice were divided into normal mice (CON) and psoriasis-induced mice (IMQ). Before inducing psoriasis, PBS (20 mg/kg) or HALN-3 (2 or 10 mg) was applied to the dorsal area of each shaved mouse twice daily at 3-hour intervals for 4 days using a brush. Three hours after drug application, 62.5 mg of Aldara cream was applied once daily for 4 days. On day 5, skin tissues were collected from all of the mice.

In the same manner as in Experimental Example 2, the PASI scores were determined, and histological staining was performed to measure the skin thickness and the expression of cell proliferation-related factors (Ki-67 and CD68) and inflammatory cytokines (IL-23 and IL-17).

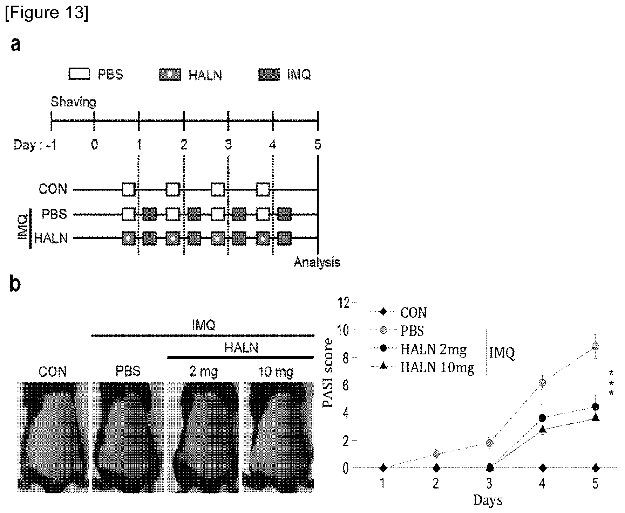

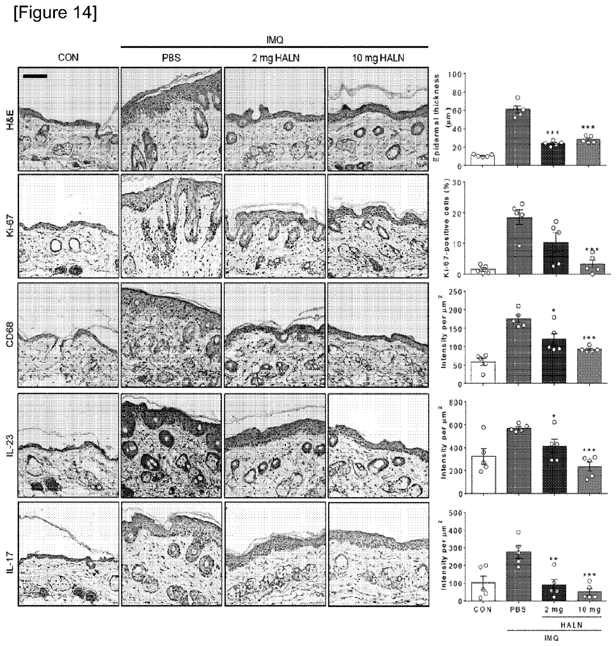

As a result, as shown in FIGS. 13 and 14, it was confirmed that, when HALN was administered, the skin condition was improved in a concentration-dependent manner, and cell proliferation-related factors and inflammatory cytokines were effectively inhibited.

These results suggest that HALN is effective in preventing skin inflammatory diseases such as psoriasis.

### Experimental Example 6. Protective effect of transdermal administration of HALN on skin barrier function

Mice were divided into normal mice (CON) and psoriasis-induced mice (IMQ). Before inducing psoriasis, PBS (20 mg/kg) or HALN-3 (2 or 10 mg) was applied to the dorsal area of each shaved mouse twice daily at 3-hour intervals for 4 days using a brush. Three hours after drug application, 62.5 mg of Aldara cream was applied once daily for 4 days. On day 5, skin tissues were collected from all of the mice. Markers (ceramide, loricrin, claudin-1, and S100A9) representing skin barrier function in skin tissues were immunostained.

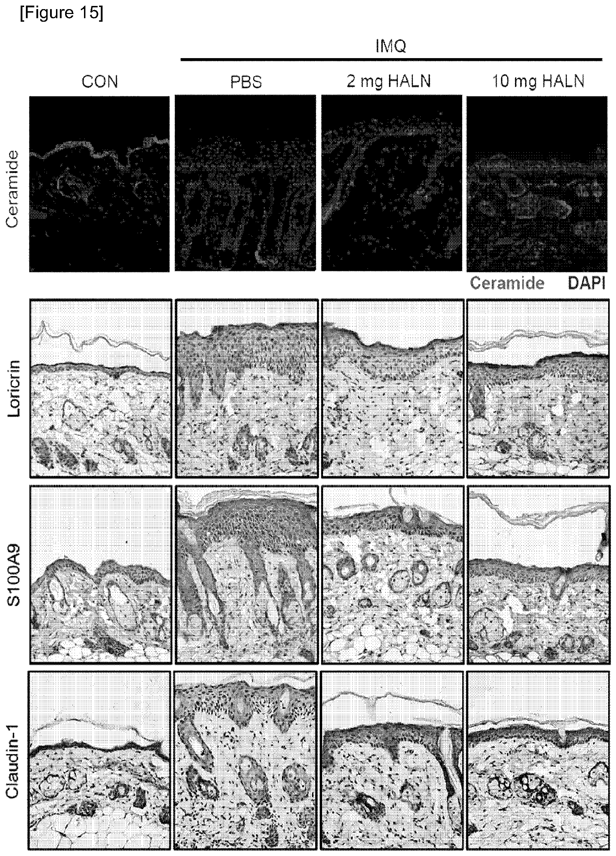

As a result, as shown in FIG. 15, it was confirmed that, when HALN-3 was administered, the skin barrier function destroyed by the induction of psoriasis was restored in a concentration-dependent manner.

### Experimental Example 7. Size-dependent effect of HALN on inhibition of macrophage polarization

Human monocyte THP-1 cells were seeded in a 6-well plate (SPL) at 5×10⁵ cells/well, treated with 50 ng/ml of PMA (Phorbol 12-myristate 13-acetate), and cultured in an incubator at 37°C under 5% CO₂ for 24 hours. Thereafter, the medium was replaced with a medium (RPMI-1640) containing 10% fetal bovine serum (FBS), and then the cells were cultured in an incubator at 37°C under 5% CO₂ for 24 hours.

Each HALN was diluted in serum-free medium (RPMI-1640) containing LPS and IFNγ to a concentration of 50 µg/ml, and THP-1 cells with 1 ml of the dilution and then cultured in an incubator at 37°C under 5% CO₂ for 4 hours. After completion of the experiment, RNA was isolated from the cells using Trizol reagent (Molecular Research Center), and then a sample with a yield of 260/280 ratio of 2.0 or higher was used for analysis using Nanodrop. From the isolated RNA, a cDNA template was synthesized with reverse transcriptase, and qPCR was performed by amplifying the cDNA template by real-time multiplexing amplification (CFX connet^{™}, Bio-Rad) in the presence of SYBR green dye using nucleotide primers specific to each gene (see Table 1 below).

**[Table 1]**

| Gene | Primer sequence (5' → 3') | | SEQ ID NO. |
|---|---|---|---|
| GAPDH | Forward (F) | TGC ACC ACC AAC TGC TTA GC | 1 |
| | Reverse (R) | GGC ATG GAC TGT GGT CAT GAG | 2 |
| TNF-α | Forward (F) | TGG GAT CAT TGC CCT GTG AG | 3 |
| | Reverse (R) | CCA GGT TTC GAA GTG GTG GT | 4 |
| IL-6 | Forward (F) | CCA CTC ACC TCT TCA GAA CG | 5 |
| | Reverse (R) | CAT CTT TGG AAG GTT CAG GTT G | 6 |
| IL-1β | Forward (F) | TTC GAC ACA TGG GAT AAC GAG G | 7 |
| | Reverse (R) | TTT TTG CTG TGA GTC CCG GAG | 8 |
| IL-12p40 | Forward (F) | AAC CCT GAC CAT CCA AGT CAA A | 9 |
| | Reverse (R) | CTT GGC CTC GCA TCT TAG AAA G | 10 |
| IL-23p19 | Forward (F) | GCT TAG AAA CTC GGT GAA CAA CT | 11 |
| | Reverse (R) | GTG GAA TCT CTG CCC ACT TCC | 12 |

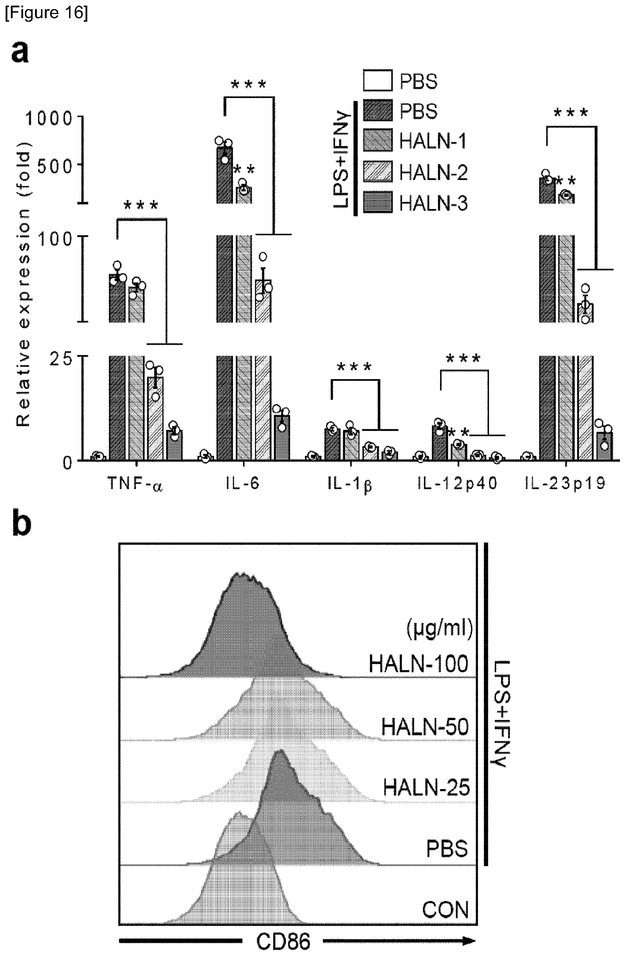

As a result, as shown in FIG. 16(a), it was confirmed that HALN-1 to 3 reduced the expression levels of all of M1 macrophage-specific genes that were increased by LPS and IFNγ, and in particular, HALN-3 with the smallest size most effectively inhibited M1 macrophage polarization.

In addition, HALN-3 was diluted in a serum-free medium (RPMI-1640) containing LPS and IFNγ to concentrations of 0, 25, 50, and 100 µg/ml, and THP-1 cells were treated with 1 ml of each dilution, and then cultured in an incubator at 37°C under 5% CO₂ for 48 hours. The treated cells were washed once with PBS, and then detached from the cell culture dish using a cell detachment solution (Accutase). Next, the cells recovered by centrifugation were placed into each FACS tube at 5×10⁵ cells and then washed once with a reaction solution (PBS, containing 5 mM EDTA, 2% FBS and 2% BSA). Thereafter, a reaction solution containing CD86, a marker for M1 macrophages, was added to the cells, followed by reaction in a dark room at 4°C for 1 hour. Next, the cells were washed three times with a reaction solution, and then 300 µl of a reaction solution was added to the cells which were then dispersed so as not to aggregate. Then, the fluorescence of APC was measured using a flow cytometer (Novocyte, Agilent).

As a result, as shown in FIG. 16(b), it was confirmed that the CD86 expression level of M1 macrophages that was increased by LPS and IFNγ decreased in a manner dependent on the concentration of HALN-3. These results suggest that HALN-3 effectively inhibits the polarization of M1 macrophages.

### Example 2. Production of hyaluronic acid-cholanic acid conjugate (HACN)

### 2-1. Amination of cholanic acid

1 g of 5β-cholanic acid (CA) (Sigma-Aldrich) was dissolved in 20 mL of methanol, and then 180 µL of HCl (12 N) was added thereto, followed by stirring at 70°C for 8 hours under reflux conditions. After completion of the reaction, the solvent was distilled off under reduced pressure, and the resulting solid product was diluted in water. The precipitate was collected by filtration under reduced pressure and dried in a vacuum to finally obtain cholanic acid (CA) methyl ester. 925 mg of the CA methyl ester was dissolved in 15 mL of ethylenediamine (TCI), followed by stirring at 130°C for 8 hours under reflux conditions. After completion of the reaction, the ethylenediamine was distilled out under reduced pressure, and the resulting product was diluted in water. Then, the precipitate was collected by filtration under reduced pressure and dried to finally obtain aminated CA.

### 2-2. Hyaluronic acid-cholanic acid conjugate (HACN)

120 mg of 10 kDa hyaluronic acid (HA; Lifecore) was dissolved in 20 mL of formamide (Sigma-Aldrich). Then, 1-ethyl-3-(3-dimethylaminopropyl)carbodiimide (TCI) and N-hydroxysuccinimide were added thereto, followed by stirring for 30 minutes, thus preparing an HA mixture. Aminated CA (29.4 mg, 0.073 mmol) according to the feed ratio (FR) value corresponding to a DS value of 3 was dissolved in 5 mL of dimethylformamide, and then this solution was added dropwise to the HA mixture. The resulting mixture was stirred at room temperature for 24 hours. After completion of the reaction, the reaction mixture was dialyzed using a 35K MWCO snakeskin dialysis bag (Thermo Scientific) in methanol alone for 2 days, a mixture of methanol: distilled water (v:v=1:1) for 1 day, and distilled water alone for 2 days. After completion of dialysis, the reaction mixture was sonicated for 50 seconds (10 seconds on, and 1 second off) . After sonication, the mixture was filtered through a 0.8-um syringe filter. The filtrate was freeze-dried to finally obtain a hyaluronic acid-cholanic acid conjugate (HACN).

### 2-3. Characterization of HACN

Chemical characterization of HACN was performed by nuclear magnetic resonance (NMR) spectroscopy at 600 MHz using deuterated methanol: deuterium oxide (1:1 (v:v)) as a solvent. The size and zeta potential of the produced HACN were measured using dynamic light scattering (DLS) (ELS-Z, Otsuka). For measurement, HACN was dissolved in PBS at a concentration of 1 mg/mL.

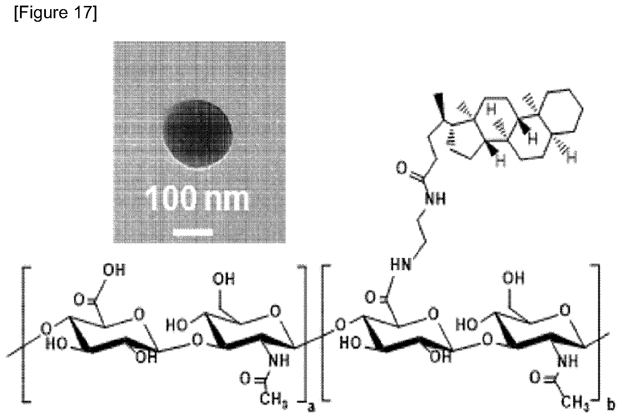

As a result, as shown in FIG. 17, when the dried HACN was dispersed in PBS, functional groups of cholanic acid aggregated together to form a spherical shape. The DS (degree of substitution of CA), size, and zeta potential (ζ) of HACN are shown in Table 2 below.

**[Table 2]**

| DS | Size (nm) | ζ (mV) |
|---|---|---|
| 3.35 | 20015 | -14.64±0.92 |

### Experimental Example 8. Analysis of transdermal permeability of HACN

After the dorsal skin from each of PBS-applied normal mice and psoriasis-induced mice was fixed to a Franz diffusion cell, Cy5.5-HACN-3 (0.3 ml) was applied onto the skin tissue. Psoriasis was induced by applying 62.5 mg of Aldara cream to the dorsal area of each shaved mouse once a day for 4 days. Skin tissues were collected 1, 4, and 12 hours after application of Cy5.5-HACN, and each tissue was fixed with 4% PFA. Then, a paraffin block was made and sectioned into 0.4-pm-thick slides, thus preparing samples for histological staining. In addition, after DAPI staining, the skin tissues were observed under a fluorescence microscope.

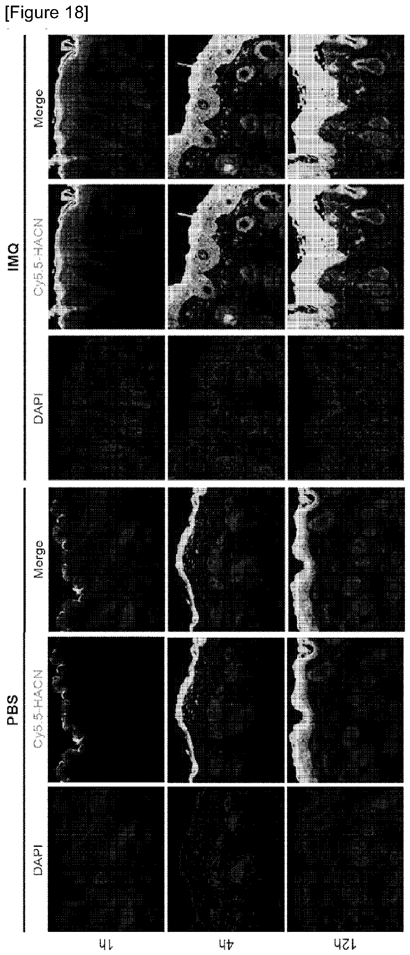

As a result, as shown in FIG. 18, it was observed that, in the skin from the normal mice (PBS), Cy5.5-HACN penetrated the skin over time. In addition, it was confirmed that the fluorescence intensity in the psoriasis-induced mice (IMQ) was stronger than that in the normal mice under the same time conditions, indicating that the transdermal permeability of HACN in the psoriasis skin was better.

### Experimental Example 9. Macrophage-dependent effect of HACN on treatment of psoriasis

Macrophage depletion was induced by the first intraperitoneal injection of 200 µl/mouse (1.4 mg) of clodronate liposomes into 7-8-week-old female C57BL/6N mice, and then the second intraperitoneal injection of 100 µl/mouse (0.7 mg) of clodronate liposomes on day 4. The next day after the first injection, the dorsal area was shaved, and after one day, 62.5 mg of Aldara cream was applied to the dorsal area once daily for 5 days to induce psoriasis skin disease models. HACN or PBS was administered by tail vein injection 2 hours before application of Aldara cream during the same period (5 mice per group). The psoriasis induction areas were imaged and compared on the first and last days of psoriasis induction, and the PASI scores of the mice were measured and compared every day. The dorsal skin was excised and fixed with 4% PFA, and then a paraffin block was made and sectioned into 4 pm-thick slides, thus preparing skin samples for histological staining. The epidermal thickness was measured through H&E staining. In addition, skin cell proliferation was measured through staining of Ki-67, and the degree of inflammatory response was measured through staining of the inflammatory cytokines IL-1β, IL-23 and IL-17.

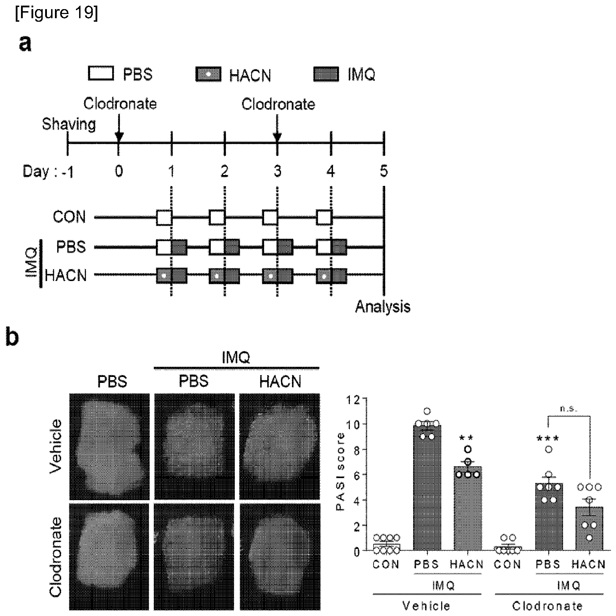

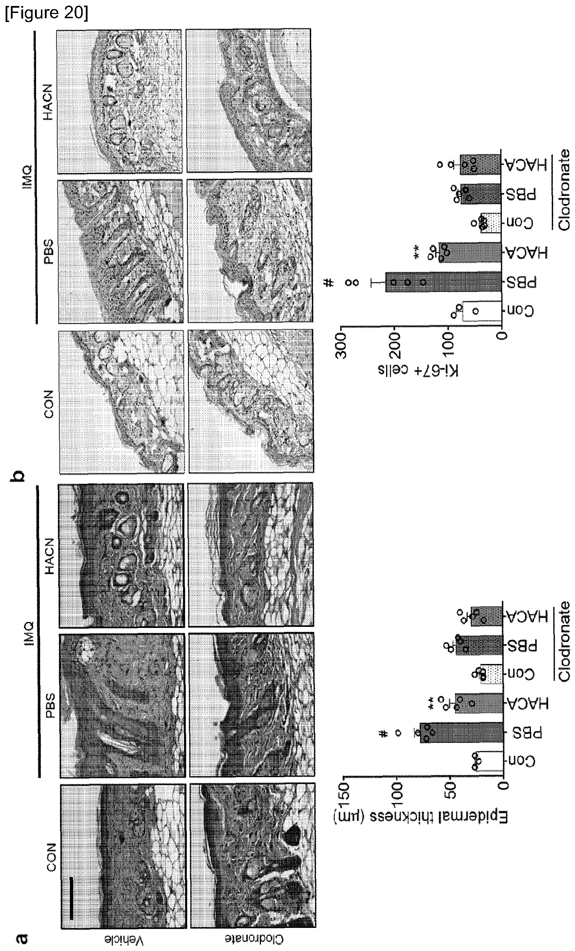

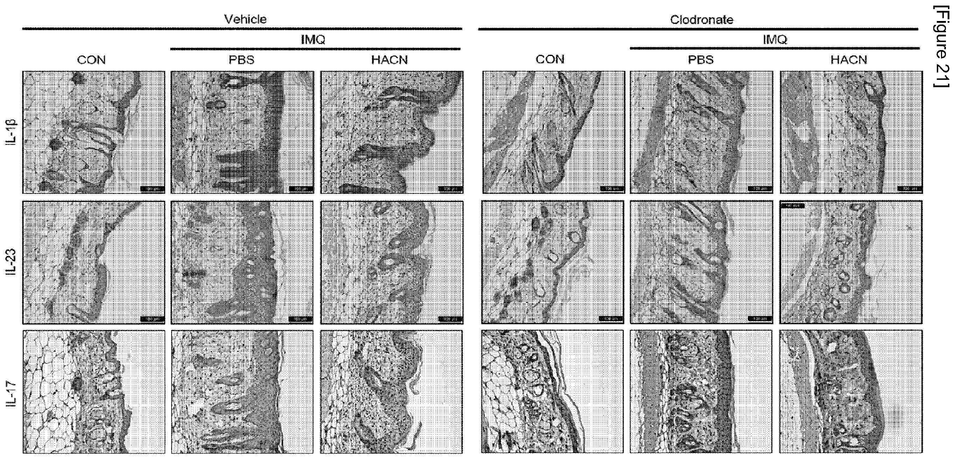

As a result, as shown in FIGS. 19 to 21, it was confirmed that, in the non-clodronate-treated group (Vehicle), HACN reduced the PASI score and epidermal thickness and inhibited skin cell proliferation and inflammatory response, indicating that HACN is effective in treating psoriasis. However, in the group treated with clodronate, the effect of HACN on the treatment of psoriasis was reduced due to macrophage depletion. These results suggest that the effect of HACN on the treatment of psoriasis is dependent on macrophages.

### Experimental Example 10. Effect of HACN on inhibition of macrophage polarization

qPCR was performed in the same manner as in Experimental Example 7, except that 0, 10, 25, and 50 ug/ml of HACN were used instead of HALN, and the primers shown in Table 3 below were used instead of the primers shown in Table 1.

**[Table 3]**

| Gene | Primer sequence (5' → 3') | | SEQ ID NO. |
|---|---|---|---|
| GAPDH | Forward (F) | TGC ACC ACC AAC TGC TTA GC | 1 |
| | Reverse (R) | GGC ATG GAC TGT GGT CAT GAG | 2 |
| TNF-α | Forward (F) | TGG GAT CAT TGC CCT GTG AG | 3 |
| | Reverse (R) | CCA GGT TTC GAA GTG GTG GT | 4 |
| IL-6 | Forward (F) | CCA CTC ACC TCT TCA GAA CG | 5 |
| | Reverse (R) | CAT CTT TGG AAG GTT CAG GTT G | 6 |
| IL-12p40 | Forward (F) | AAC CCT GAC CAT CCA AGT CAA A | 9 |
| | Reverse (R) | CTT GGC CTC GCA TCT TAG AAA G | 10 |
| IL-23p19 | Forward (F) | GCT TAG AAA CTC GGT GAA CAA CT | 11 |
| | Reverse (R) | GTG GAA TCT CTG CCC ACT TCC | 12 |
| COX-2 | Forward (F) | ATG CTG ACT ATG GCT ACA AAA GC | 13 |
| | Reverse (R) | TCG GGC AAT CAT CAG GCA C | 14 |
| CXCL10 | Forward (F) | GAA AGC AGT TAG CAA GGA AAG GTC | 15 |
| | Reverse (R) | ATG TAG GGA AGT GAT GGG AGA GG | 16 |

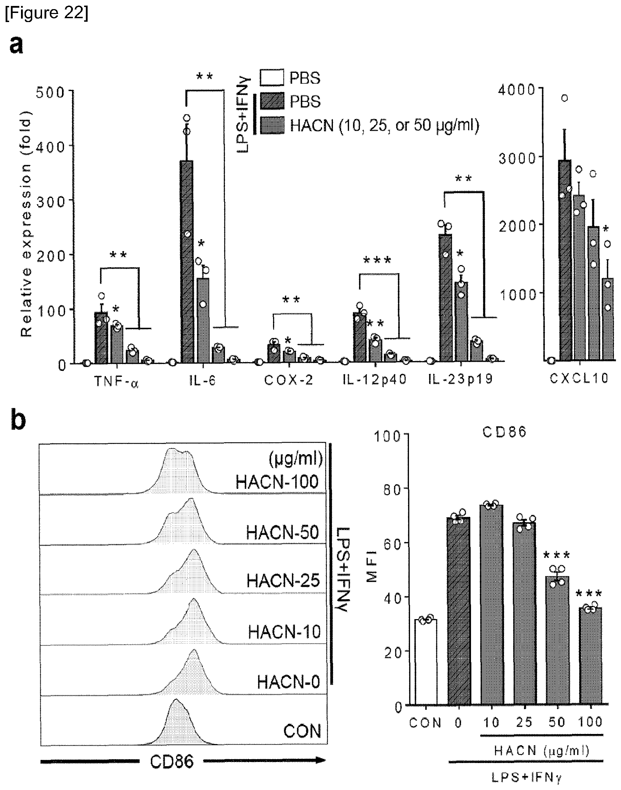

As a result, as shown in FIG. 22(a), it was confirmed that HACN decreased the expression of all M1 macrophage-specific genes, which was increased by LPS and IFNγ, in a concentration-dependent manner, suggesting that HACN inhibited the polarization of THP-1 macrophages.

In addition, APC fluorescence was measured in the same manner as in Experimental Example 7, except that 0, 10, 25, and 50 ug/ml of HACN were used instead of HALN.

**As** a result, as shown in FIG. 22(b), it was confirmed that the CD86 expression level of M1 macrophages, which was increased by LPS and IFNγ, decreased in a manner dependent on the concentration of HACN. These results suggest that HACN effectively inhibits the polarization of M1 macrophages.

### Experimental Example 11. Analysis of interaction between HACN and TLR4

### 11-1. Surface plasmon resonance (SPR)

In order to immobilize a target receptor on an assay chip, the receptor was immobilized on the chip by flowing recombinant TLR4 while flowing an EDC/NHS solvent. Thereafter, ethanolamine was flowed to remove weakly immobilized or non-immobilized receptor on the chip. While each of PBS, HACN in PBS as a solvent, and 10 kDa free HA in PBS as a solvent was flowed on the resulting recombinant TLR4-immobilized chip at concentrations of 0, 25, 50, 100, 200, 400, and 800 µg/ml and at a rate of 10 ul/min, a change in the response unit (RU) value was observed for 120 seconds.

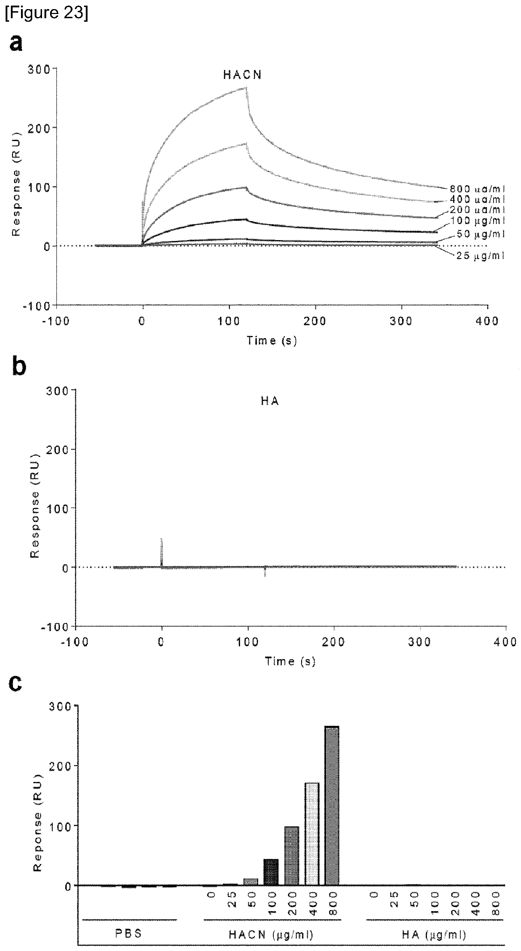

As a result, as shown in FIG. 23, it could be confirmed that the RU value increased as the concentration of HACN increased, suggesting that TLR4 and HACN interacted with each other. On the other hand, in the control group on which only PBS or 10 kDa free HA was flowed, the increase in RU value identified in the HACN group was not observed. Therefore, it can be seen that binding to TLR4 is a unique feature of HACN.

### 11-2. Fluorescence cytometry

THP-1 cells were seeded into a 35-mm cell culture dish (SPL) at a density of 5×10⁵ cells, treated with 50 ng/ml of PMA, and cultured in an incubator at 37°C under 5% CO₂ for 24 hours. Thereafter, the medium was replaced with a medium (RPMI-1640) containing 10% FBS, and then the cells were further cultured in an incubator at 37°C under 5% CO₂ for 24 hours. Each of HACN and 10 kDa free HA was diluted in serum-free medium (RPMI-1640) to concentrations of 0, 10, 25, and 50 ug/ml. The cells were treated with 1 ml of each prepared dilution, and then cultured in an incubator at 37°C under 5% CO₂ for 1 hour. Thereafter, the cell culture was additionally treated with fluorescent Cy5.5-labeled LPS and 1 ml of HACN, and then further cultured in an incubator at 37°C under 5% CO₂ for 30 minutes. After completion of the experiment, the cells were washed with cold PBS and then fixed with 4% PFA. The fixed cells were analyzed for tendency using a fluorescence microscope.

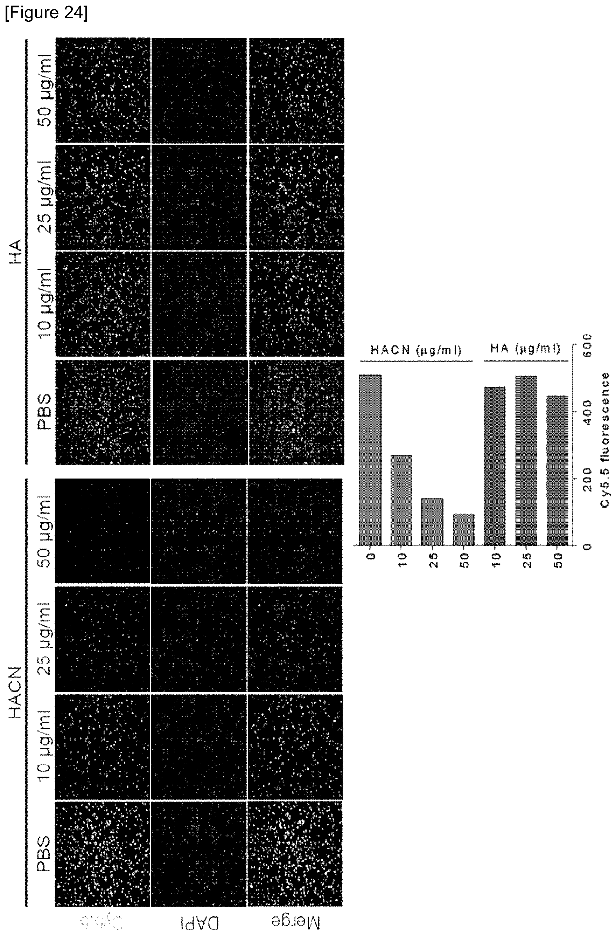

As a result, as shown in FIG. 24, it was confirmed that the binding of Cy5.5-labeled LPS in the THP-1 macrophages decreased in a manner dependent on the concentration of HACN, but was not decreased by 10 kDa free HA. These results suggest that HACN inhibits LPS-induced TLR4 activation by binding to TLR4 competitively with LPS.

### Example 3. Production of hyaluronic acid-cholesterol conjugate (HACHN)

### 3-1. Amination of cholesterol

1 g of cholesterol (Sigma Aldrich) 1 g, 544 mg of N-N-Boc-glycine (Alfa Aesar), 640 mg of N,N'-dicyclohexylcarbodiimide (DCC; Sigma Aldrich), and 47.4 mg of 4-(dimethylamino)pyridine (DMAP; Alfa Aesar) were dissolved in 25 mL of dichloromethane, followed by stirring at 25°C for 24 hours. After completion of the reaction, the reaction mixture was dried in a vacuum and separated by liquid chromatography (hexane: ethyl acetate = 1:10). The resulting material was dried to finally obtain Boc-Gly-cholesterol. Subsequently, 1.09 g of Boc-Gly-cholesterol was dissolved in 20 mL of dichloromethane, and 5 mL of a 4N hydrochloric acid/1,4-dioxane solution was added thereto, followed by stirring at 25°C for 1 hour. After completion of the reaction, the reaction product was dried in a vacuum to finally obtain aminated cholesterol.

### 3-2. Hyaluronic acid-cholesterol conjugate (HACHN)

120 mg of 10 kDa hyaluronic acid (HA; Lifecore Biomedical) was dissolved in 20 mL of formamide (Sigma-Aldrich). Then, 1-ethyl-3-(3-dimethylaminopropyl)carbodiimide (TCI) and N-hydroxysuccinimide (Sigma Aldrich) were added thereto, followed by stirring for 20 minutes, thus preparing an HA mixture. An amount of aminated cholesterol (70.1 mg, 0.146 mmol) according to the feed ratio (FR) value corresponding to a DS value of 2 was dissolved in 5 mL of dimethylformamide together with triethylamine (TCI), and then this solution was added dropwise to the HA mixture. The resulting mixture was stirred at room temperature for 24 hours. After completion of the reaction, the reaction mixture was dialyzed using a 3.5K MWCO snakeskin dialysis bag (Thermo Scientific) in methanol alone for 2 days, a mixture of methanol: distilled water (v:v=1:1) for 1 day, and distilled water alone for 2 days. After completion of dialysis, the reaction mixture was sonicated for 50 seconds (10 seconds on, and 1 second off). After sonication, the mixture was filtered through a 0.8-um syringe filter. The filtrate was freeze-dried to finally obtain a hyaluronic acid-cholesterol conjugate (HACHN).

### 3-3. Characterization of HACHN

¹H nuclear magnetic resonance (NMR) spectra of HACHN were obtained using a nuclear magnetic resonance spectrometer (JNM-ECZ 600R, JEOL) at 600 MHz using deuterated methanol (METHANOL-D₆): deuterium oxide (1:1 (v:v)) as a solvent. The size and zeta potential of the produced HACHN were measured using dynamic light scattering (DLS) (ELS-Z, Otsuka). For measurement, HACHN was dissolved in PBS at a concentration of 1 mg/mL. As a result, when the dried HACHN was dispersed in PBS, functional groups of cholesterol aggregated together to form a spherical shape. The DS (degree of substitution of cholesterol), size, and zeta potential (ζ) of HACHN are shown in Table 4 below.

**[Table 4]**

| | DS | Size (nm) | PDI | ζ (mV) |
|---|---|---|---|---|
| HACHN | 2.34 | 25014 | 0.239±0.036 | -17.84±1.70 |

### Experimental Example 12. Analysis of skin disease treatment effects of various types of hyaluronic acid conjugates including HACHN

Twenty-eight female C57BL/6N mice were divided into normal mice (CON) and psoriasis-induced mice (IMQ). As reagents, Aldara cream, PBS, HALN, 10k-HALN, 10k-HACN, and 10k-HACHN were prepared as follows.
HALN: hyaluronic acid with molecular weight of 60 kDa + lithocholic acid
10k-HALN: hyaluronic acid with molecular weight of 10 kDa + lithocholic acid
10k-HACN: hyaluronic acid with molecular weight of 10 kDa + cholanic acid
10k-HACHN: hyaluronic acid with molecular weight of 10 kDa + cholesterol

In order to induce psoriasis, 62.5 mg of Aldara cream was applied to each mouse of each group except for the control group (CON) once daily for 4 days. Six hours before applying Aldara cream, the corresponding drug was applied to the dorsal skin of each mouse of each group using a brush, and the HA nanoparticle conjugate was dissolved in PBS and then 2 mg of the HA nanoparticle conjugate was applied. On day 5, skin tissues were collected from all of the mice and analyzed.

As an analysis method, the Psoriasis Area and Severity Index (PASI) scoring method based on the following criteria was used: 0, none; 1, slight; 2, moderate; 3, substantial; and 4, very well pronounced. In a histological staining method, each tissue was fixed, embedded, sectioned into 0.4-um-thick slices, and then stained and analyzed using hematoxylin, eosin, a DAB staining kit, and antibodies. In an intensity measurement method, analysis was performed using the [Image J] program and the Leica DMi8 fluorescence microscope, and the average of three partial image values of each tissue was used.

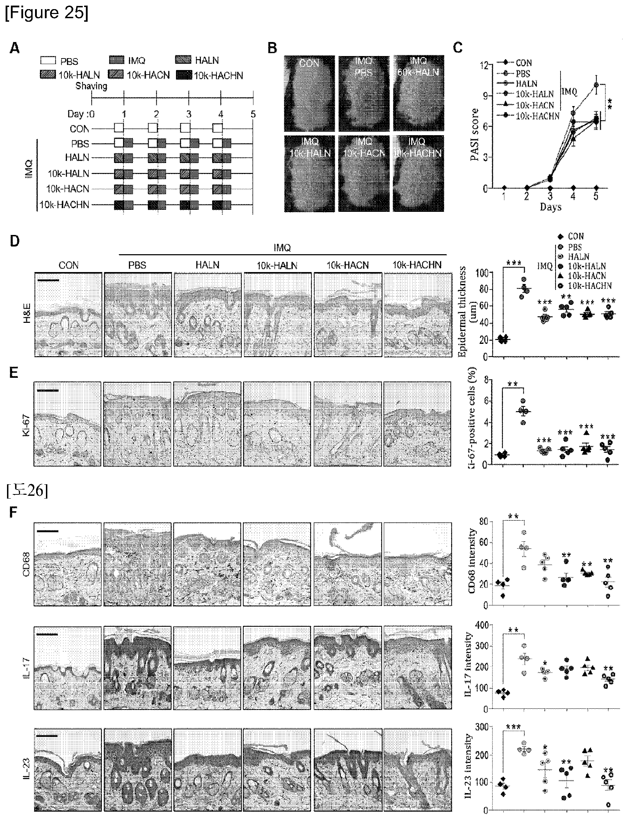

As a result, as shown in FIGS. 25 and 26, it was confirmed that the hyaluronic acid conjugates comprising different types of hydrophobic substances bound to hyaluronic acid all reduced the PASI score, skin thickness, skin cell proliferation, and inflammatory response in the group to which each of the hyaluronic acid conjugates was applied, indicating that the hyaluronic acid conjugates are effective against skin diseases.

Taken together, it can be seen that the hyaluronic acid conjugate according to the present invention, in which hyaluronic acid and a hydrophobic substance are linked together, has strong resistance to hyaluronidase and excellent transdermal permeability, and in particular, as the size of the conjugate decreases, the conjugate has better transdermal permeability, and better effects on the protection or restoration of skin barrier function, the inhibition of expression of cell proliferation-related factors and inflammatory cytokines, the inhibition of M1 macrophage polarization, and the blocking of TLR4 signaling, and thus is more effective in preventing or treating various skin diseases such as psoriasis, malignant melanoma, bacterial and fungal inflammation, and autoimmune diseases.

So far, the present invention has been described with reference to the embodiments. Those of ordinary skill in the art to which the present invention pertains will appreciate that the present invention may be embodied in modified forms without departing from the essential characteristics of the present invention. Therefore, the disclosed embodiments should be considered from an illustrative point of view, not from a restrictive point of view. The scope of the present invention is defined by the claims rather than the foregoing description, and all differences within the scope equivalent thereto should be construed as being included in the present invention.

### Industrial Applicability

The present invention is directed to a pharmaceutical composition for preventing or treating skin diseases comprising a hyaluronic acid conjugate as an active ingredient. The pharmaceutical composition is industrially applicable because it may be used as a therapeutic agent for various skin diseases.

## Claims

1. A pharmaceutical composition for preventing or treating skin disease comprising, as an active ingredient, a hyaluronic acid conjugate composed of hyaluronic acid and a hydrophobic substance.

2. The pharmaceutical composition according to claim 1, wherein the hydrophobic substance has a partition coefficient (cLogP) of 2 to 10.

3. The pharmaceutical composition according to claim 1, wherein the hyaluronic acid conjugate is a compound represented by the following Formula 1, Formula 2, or Formula 3 : 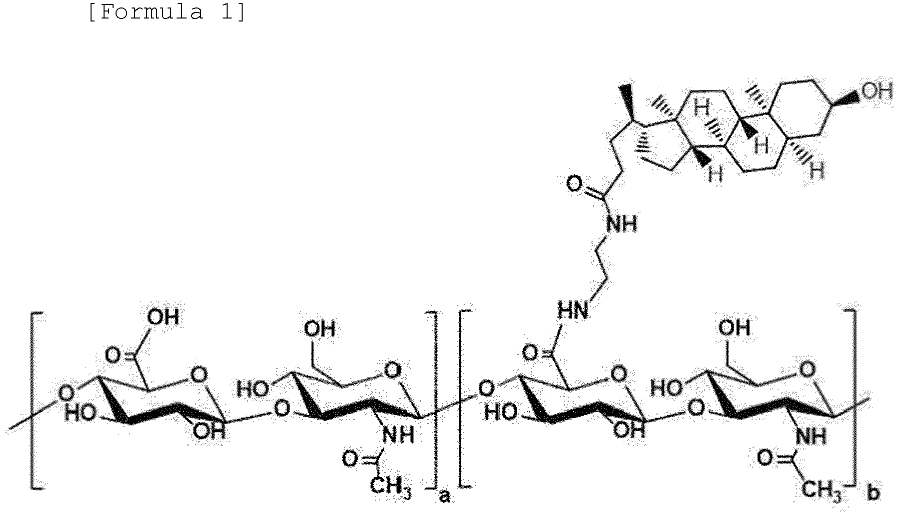
wherein a and b are independent integers that are the same as or different from each other, a is an integer ranging from 1 to 750, b is an integer ranging from 1 to 100, and b/(a+b) is in the range of 0.01 to 0.40; 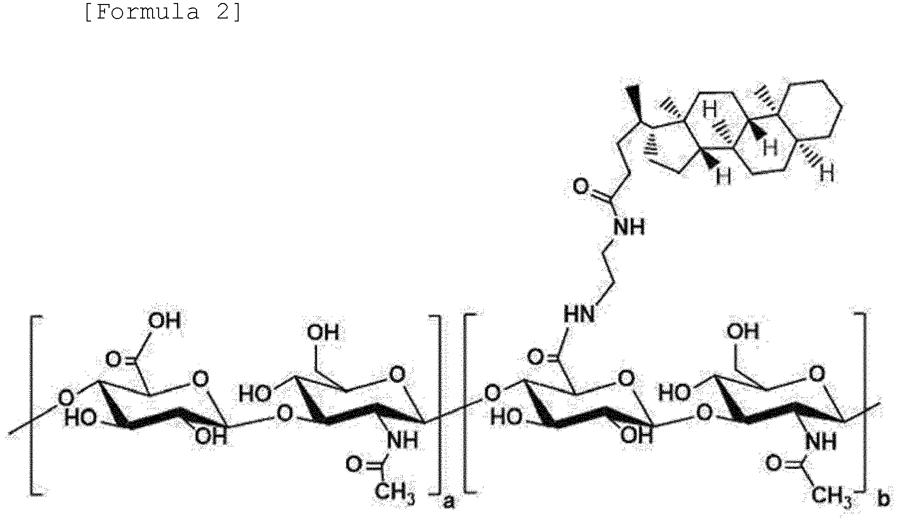
wherein a and b are independent integers that are the same as or different from each other, a is an integer ranging from 1 to 750, b is an integer ranging from 1 to 100, and b/(a+b) is in the range of 0.01 to 0.40; 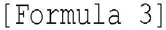 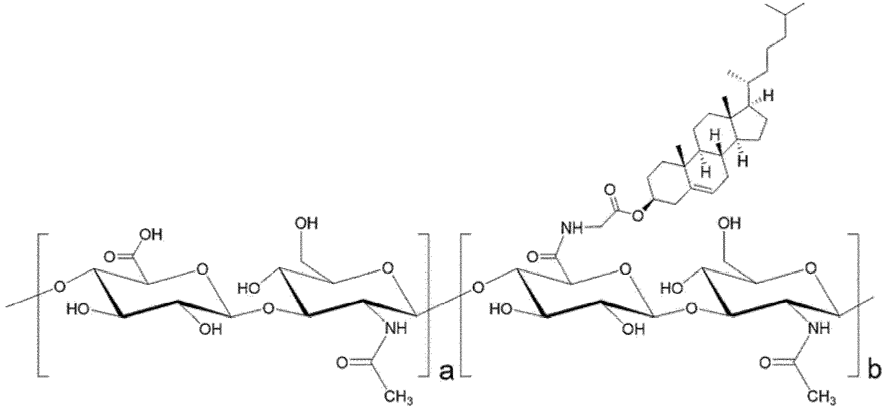
wherein a and b are independent integers that are the same as or different from each other, a is an integer ranging from 1 to 750, and b is an integer ranging from 1 to 100, and b/(a+b) is in the range of 0.01 to 0.40.

4. The pharmaceutical composition according to claim 1, wherein a mass ratio of hyaluronic acid to the hydrophobic substance in the hyaluronic acid conjugate is 1:0.005 to 0.5.

5. The pharmaceutical composition according to claim 1, wherein the hyaluronic acid conjugate is formed by self-assembly in an aqueous solution.

6. The pharmaceutical composition according to claim 1, wherein the hyaluronic acid conjugate has a diameter of 50 to 500 nm.

7. The pharmaceutical composition according to claim 1, wherein the hyaluronic acid conjugate is for transdermal administration.

8. The pharmaceutical composition according to claim 1, wherein the skin disease is at least one selected from the group consisting of atopic dermatitis, acne, psoriasis, allergic dermatitis, inflammatory skin disease, seborrheic dermatitis, contact dermatitis, scleroderma, eczema, Behcet's disease, sarcoidosis, melanoma, vitiligo, pemphigus, corns, warts, and lichen planus.

9. A method for preventing or treating skin disease comprising a step of administering to a subject a composition comprising, as an active ingredient, a hyaluronic acid conjugate composed of hyaluronic acid and a hydrophobic substance.

10. Use of a hyaluronic acid conjugate composed of hyaluronic acid and a hydrophobic substance for prevention or treatment of skin disease.

11. Use of a hyaluronic acid conjugate composed of hyaluronic acid and a hydrophobic substance for manufacture of a medicament for prevention or treatment of skin disease.
